# EUROPEAN PATENT APPLICATION

(11) **EP 4 129 253 A1**
(43) Date of publication of application: **08.02.2023**
(21) Application number: 21774823.5
(22) Date of filing: 03.02.2021
(51) Int. Cl.: A61F 13/15, A61F 13/511, A61F 13/536

(54) **ABSORBENT ARTICLE AND PACKAGED ABSORBENT ARTICLE**

(30) Priority: 26.03.2020 JP 2020055838
(71) Applicant: DAIO PAPER CORPORATION, Shikokuchuo-shi Ehime 799-0492 (JP)
(72) Inventor: KONAWA, Satoko, Sakura-shi, Tochigi 329-1411 (JP)
(74) Representative: Epping - Hermann - Fischer
(86) International application number: PCT/JP2021/003904
(87) International publication number: WO 2021/192640

(57) **Abstract**

An absorbent article includes: a liquid-impermeable outer surface sheet; an absorber; and a liquid-permeable inner surface sheet. These are laminated in the order named. The absorbent article has a longitudinal direction that corresponds to the longitudinal direction of the body of the user of the absorbent article, and a width direction that is orthogonal to the longitudinal direction. The absorbent article has a middle area that includes a body fluid discharging orifice facing area facing a body fluid discharging orifice in the body of the person, a front area that is joined to a front portion of the middle area, and a rear area that is joined to a rear portion of the middle area. A chemical-carrying portion is formed in at least one of the front area and the rear area of the absorbent article, the chemical-carrying portion being where a chemical is applied to a surface that is separate from an exposed surface of the absorbent article. A compressed portion is provided at a distance from the chemical-carrying portion, on a straight line that connects the center of the body fluid discharging orifice facing area and the chemical-carrying portion.

## Description

### TECHNICAL FIELD

The present invention relates to an absorbent article and a wrapped absorbent article.

### BACKGROUND ART

Absorbent articles such as sanitary napkins, panty liners, incontinence pads, and the like, to which a chemical containing a fragrance element, a deodorant element, or any other functional element is imparted, are known.

For example, Patent Document 1 discloses an absorbent article, in which a fragrance is provided inside a portion of an absorber located so as to face an excretory part, and in which multiple slits are formed in the excretory part-facing portion and reach the surface where the fragrance is provided. Patent Document 1 describes, as an effect of the above configuration, that the fragrance is scented continuously and optimally.

### RELATED-ART DOCUMENTS

### PATENT DOCUMENTS

Patent Document 1: Japanese Unexamined Patent Application Publication No. 2017-217

### SUMMARY OF THE INVENTION

### PROBLEM TO BE SOLVED BY THE INVENTION

However, as is the case with the absorbent article described in Patent Document 1, in a configuration in which a fragrance is provided in a portion of an absorber facing an excretory part, it necessarily follows that most of the fragrance comes into contact with a body fluid during use. Depending on the type of the fragrance, the function of the fragrance might deteriorate by contacting the body fluid. There is a possibility that chemicals other than fragrances might suffer this deterioration of function.

One aspect of the present invention is therefore intended to provide an absorbent article that is capable of optimally exerting the function of a chemical such as a fragrance even during use.

### MEANS TO SOLVE THE PROBLEM

One aspect of the present invention provides an absorbent article that includes: a liquid-impermeable outer surface sheet; an absorber; and a liquid-permeable inner surface sheet. These are laminated in the order named. The absorbent article has a longitudinal direction that corresponds to the longitudinal direction of the body of the user of the absorbent article, and a width direction that is orthogonal to the longitudinal direction. The absorbent article has a middle area that includes a body fluid discharging orifice facing area facing a body fluid discharging orifice in the body of the person, a front area that is joined to a front portion of the middle area, and a rear area that is joined to a rear portion of the middle area. A chemical-carrying portion is formed in at least one of the front area and the rear area of the absorbent article, the chemical-carrying portion being where a chemical is applied to a surface that is separate from an exposed surface of the absorbent article. A compressed portion is provided at a distance from the chemical-carrying portion, on a straight line that connects the center of the body fluid discharging orifice facing area and the chemical-carrying portion.

### EFFECTS OF THE INVENTION

According to one aspect of the present invention, it is possible to provide an absorbent article that is capable of optimally exerting the function of a chemical such as a fragrance even during use.

### BRIEF DESCRIPTION OF THE DRAWINGS

FIG. 1 is a plan view of an absorbent article according to one example of the present invention;
FIG. 2 is a cross-sectional view taken along line I-I in FIG. 1;
FIG. 3 is a plan view of an absorbent article according to a modification of the present invention;
FIG. 4 is a plan view of an absorbent article according to another modification of the present invention;
FIG. 5 is a plan view of an absorbent article according to yet another modification of the present invention;
FIG. 6 is a plan view of an absorbent article according to yet another modification of the present invention;
FIG. 7 is a plan view of an absorbent article according to yet another modification of the present invention;
FIG. 8 is a plan view of an absorber used in the absorbent article illustrated in FIG. 1;
FIG. 9 is a plan view of an absorbent article according to yet another modification of the present invention;
FIG. 10 is a plan view of an absorber used in the absorbent article illustrated in FIG. 9; and
FIG. 11 is a plan view of a wrapped absorbent article, in which the absorbent article illustrated in FIG. 1 is folded and wrapped with a wrapping sheet.

### MODE FOR CARRYING OUT THE INVENTION

Below, an embodiment of the present invention will be described in detail with reference to the accompanying drawings. Note that, in the following drawings, unless explained otherwise, the same or corresponding components might be assigned the same reference signs and the description thereof might be omitted. In addition, the following drawings are simply schematic ones to help understand the present invention.

### (Basic structure of the absorbent article)

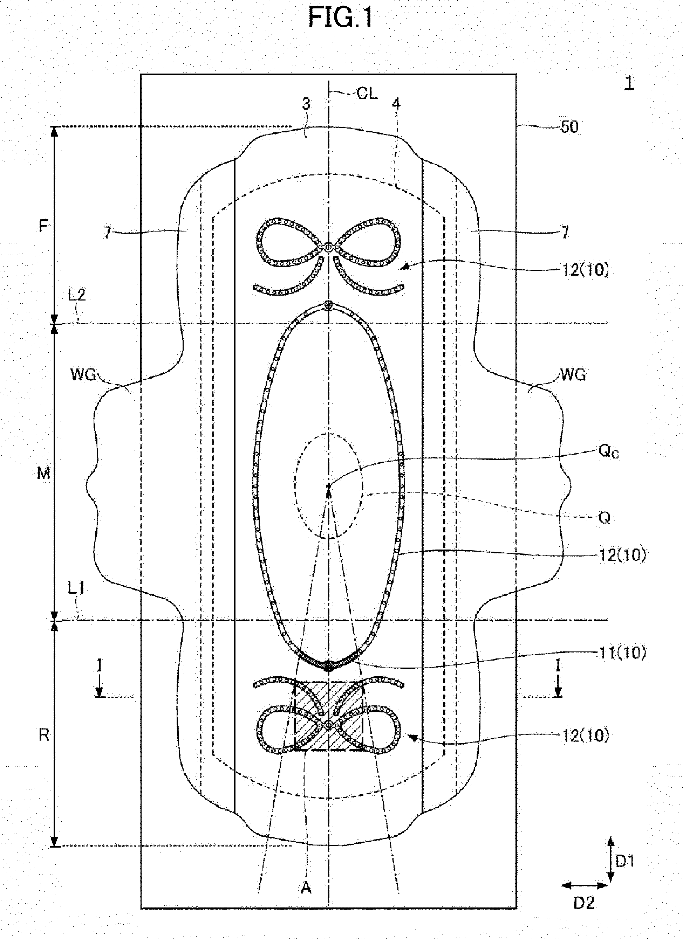

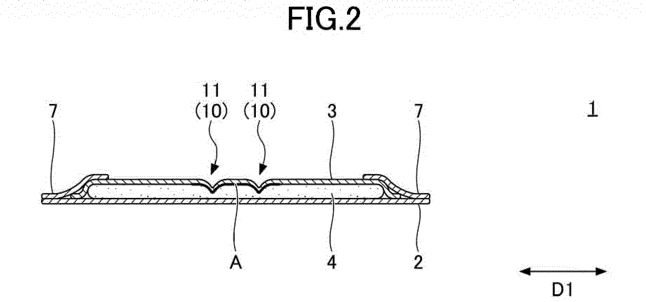

First, the basic structure of the absorbent article will be described by using a sanitary napkin as an example. FIG. 1 illustrates a plan view of an absorbent article 1 according to one example of the present invention. Furthermore, FIG. 2 shows a cross-sectional view of the absorbent article 1 taken along line I-I. As illustrated in FIG. 1 and FIG. 2, the absorbent article 1 is composed of a liquid-impermeable outer surface sheet 2, an absorber 4, and a liquid-permeable inner surface sheet 3, laminated in the order named, and has a flat shape on the whole. When the absorbent article 1 is in use, the inner surface sheet 3 is the side that comes into contact with the skin (hence "the skin side" or "the inner side"), and the outer surface sheet 2 is the side that is fixed to the underwear (hence "the underwear side" or "the outer side").

In the present specification, as illustrated in FIG. 1, the absorbent article 1 has a longitudinal direction D1 and a width direction D2. The longitudinal direction D1 of the absorbent article 1 refers to the front side and the rear side of the body of the person wearing the absorbent article 1. The width direction D2 is orthogonal to the longitudinal direction D1. In the example illustrated in FIG. 1, the absorbent article 1 has a plan-view shape that is substantially line-symmetrical with respect to a center line CL that extends in the longitudinal direction D1 (the longitudinal center line), but this shape does not have to be line-symmetrical. Furthermore, configurations of the absorbent article 1 apart from the shape (such as the density and material of the absorber, the size and locations of compressed portions, etc.) may be substantially symmetrical or asymmetrical with respect to the center line CL as an axis of symmetry.

The absorbent article 1 has: a middle area M, which primarily faces the crotch of the person wearing the absorbent article 1; a front area F, which is an area adjoining the front of the middle area M and ranges up to the frontal end of the absorbent article 1; and a rear area R, which adjoins the rear of the middle area M and ranges up to the rear end of the absorbent article 1. The middle area M includes a body fluid discharging orifice facing area Q. The body fluid discharging orifice facing area Q is an area to face the body fluid discharging orifices of the person wearing the absorbent article 1, such as the urethral opening, the vaginal opening, and the like. Its center Q_{C} is located on the longitudinal center line CL. The length of the body fluid discharging orifice facing area (the length in the first direction D1) may be approximately 10 mm to 50 mm, and the width (the length in the second direction D2) may be approximately 5 mm to 40 mm. Although FIG. 1 illustrates the body fluid discharging orifice facing area Q as an elliptical area to face the vaginal opening, the size and shape of the body fluid discharging orifice facing area Q illustrated is simply an example for describing the absorbent article according to the present embodiment.

When the absorbent article 1 is folded in three or more and wrapped individually, the front area F is folded back to the inner surface sheet 3 side at a fold line that runs along the width direction D2, and piled on the middle area M. Similarly, when the absorbent article 1 is folded in three or more and wrapped individually, the rear area R is folded back to the inner surface sheet 3 side at another fold line that runs along the width direction D2, and piled on the middle area M. When the length of the entire absorbent article 1 is long, the rear area R might be folded along yet another fold line that runs along the width direction D2 in the rear area R, before being piled on the middle area M. Note that the middle area M may be an area to include wings WG and WG across the longitudinal direction D, and correspond to an area from the location of the front starting point of the wings WG and WG to the location of the rear starting point.

As illustrated in FIG. 1, the wings WG and WG are portions of the middle area M that protrude outwards in the second direction D2. Adhesive portions are formed on the outer surface sheet 2 side of the wings WG and WG, so that the wings WG and WG can be folded back to the outer surface sheet 2 side and fixed to the underwear, ensuring that the absorbent article 1 is fixed to the underwear.

The outer surface sheet 2 may be a sheet that is at least water-impermeable. For example, a sheet made of an olefin resin such as polyethylene or polypropylene may be used. In addition, it is also possible to use a laminated non-woven fabric, in which a non-woven fabric is laminated over a polyethylene sheet or the like, a laminated sheet of a non-woven fabric, in which a waterproof film is additionally interposed so as to substantially ensure liquid impermeability, and so forth. Furthermore, from the viewpoint of preventing stuffiness, it is even more desirable to use a material that is moisture-permeable. As an example of a water-impervious/breathable sheet material like the above, a microporous sheet that is obtained by forming a sheet by melting and kneading an inorganic filler in an olefin resin such as polyethylene or polypropylene and then stretching this sheet uniaxially or biaxially can be used.

The inner surface sheet 3 can be made a liquid-permeable sheet that body fluids such as menstrual blood, vaginal discharge, and urine permeate fast. For the inner surface sheet 3, a perforated or non-perforated non-woven fabric, a porous plastic sheet, or the like is suitable for use. As for the material fiber to constitute the non-woven fabric, for example, synthetic fiber such as polyesters, polyamides, olefins including polyethylene and polypropylene, and the like, recycled fiber such as rayon and cupra, blended fiber of these, and natural fiber such as cotton cam be used. These fibers may be each used alone, or two or more of types of these fibers can be combined and used. Examples of the non-woven fabric processing method include a spun lacing method, a spun bonding method, a thermal bonding method, a melt blown method, a needle punching method, and the like. Of these processing methods, the spun lacing method is preferable in that it can produce a non-woven fabric having excellent flexibility, the spun bonding method is preferable in that it can produce a non-woven fabric having excellent drape, and the thermal bonding method is preferable in that it can produce a luxurious and soft non-woven fabric. Furthermore, composite fiber such as core-sheath fiber formed with fiber having a high melting point as the core and fiber having a low melting point fiber as the sheath, side-by-side fiber, split fiber, and the like can also be used.

The absorber 4 may be made of any material as long as the material can absorb and hold body fluids, and preferably includes a cotton-like pulp and a water-absorbent polymer. For the water-absorbent polymer, a super-absorbent polymer (SAP), super-absorbent fiber (SAF), or a combination of these can be used. Examples of the pulp include ones composed of cellulose fiber such as a chemical pulp and a dissolving pulp obtained from wood, artificial cellulose fiber such as rayon and acetate, and the like. For the raw material of the chemical pulp, hardwood, softwood, and so forth may be used, but softwood is more suitable for use because of their long fiber length and the like.

Furthermore, synthetic fiber may be mixed with the absorber 4. For the synthetic fiber, polyolefins such as polyethylene and polypropylene, polyesters such as polyethylene terephthalate and polybutylene terephthalate, polyamides such as nylon, and copolymers of these can be used. Also, two of these can be mixed and used. Furthermore, composite fiber such as core-sheath fiber formed with fiber having a high melting point as the core and fiber having a low melting point fiber as the sheath, side-by-side fiber, split fiber, and the like can also be used. Note that it is also possible to use hydrophobic fiber subjected to surface treatment using a hydrophilizing agent and thus given an affinity for body fluids. The absorber 4 is preferably produced by fiber stacking or by an air-laid method.

At both edges of the absorber 4 in the longitudinal direction D1, an edge portion of the outer surface sheet 2 and an edge portion of the inner surface sheet 3 are joined by an adhesive, heat sealing, or the like. Furthermore, in both side portions of the absorbent article 1, that is, on both sides of the absorbent article 1 in the width direction D2, side non-woven fabrics 7 and 7 are provided on the front side (inner surface sheet 3 side) in the longitudinal direction D1. The side non-woven fabrics 7 and 7 protrude outwards in the second direction D2 in the middle area M, and are joined with the outer surface sheet 2, which similarly protrude outwards in the second direction D2 in the middle area M, thereby forming the above-noted wings WG and WG. Note that the absorbent article 1 may be configured without the wings WG and WG as well.

The side non-woven fabrics 7 may be configured by using a non-woven fabric material subjected to an appropriate water-repellent treatment or a hydrophilic treatment, depending on purpose such as preventing the permeation of body fluids, enhancing the feeling of touch, and the like. The material of the side non-woven fabrics 7 may be natural fiber, synthetic fiber, recycled fiber, and the like. When applying a water-repellent treatment to the side non-woven fabric 7, a silicon-based water-repellent agent, a paraffin-based water-repellent agent, or other water-repellent agents can be used for the treatment. Note that the absorbent article may be configured such that the inner surface sheet 3 extends to the end portion of the absorbent article 1 in the width direction D2 and is joined with the outer surface sheet 2, without using the side non-woven fabrics 7.

The thickness of the absorber 4 can be preferably 0.3 mm to 30 mm, more preferably 1.0 mm to 15 mm. It should be noted that the thickness of the absorber 4 does not have to be uniform over the entire surface of the absorber 4, and may be configured such that the body fluid discharging orifice facing area Q and its nearby areas are swollen. Also, the length of the absorber 4 in the longitudinal direction (lengthwise direction) D1 is preferably 110 mm to 450 mm, more preferably 130 mm to 290 mm. Furthermore, the length of the absorber 4 in the width direction (short direction) D2 can be 40 mm to 200 mm, preferably 60 mm to 120 mm. The width of the absorber 4 may be constant.

The absorber 4 may be configured by wrapping the main body portion of the absorber 4 with a wrapping sheet made of crepe paper, a non-woven fabric, or the like. Providing the absorber 4 with the wrapping sheet makes it possible to prevent the absorber 4 from twisting or cracking, and hold its shape. For the wrapping sheet, uncolored (that is, white) crepe paper or a non-woven fabric can be used, or a colored sheet can be used. When a colored wrapping sheet is used, the color of the wrapping sheet may be reflected on the inner surface sheet 3 side as a thin color. Then, when a body fluid is discharged, its color can be blended with that reflected color of the wrapping sheet and made inconspicuous. The wrapping sheet may be colored, for example, in a complementary color of the color of the body fluid. For example, in the event the absorbent article (sanitary napkin, etc.) is configured for the purpose of absorbing menstrual blood, the wrapping sheet may be colored green, which is a complementary color of red.

### (Chemical)

The absorbent article 1 may be provided with a chemical-carrying portion A where a chemical is applied. The chemical-carrying portion A includes a portion where the chemical is actually applied, and a portion where the chemical is present as a result of the chemical's permeation and diffusion from the portion where the chemical is actually applied. The chemical-carrying portion A may be provided in a place separate from the exposed surfaces of the absorbent article 1. In the present specification, the exposed surfaces of the absorbent article 1 refer to the surfaces of the absorbent article 1 that are exposed to the outside when the absorbent article 1 is unfolded, and include the skin-side surface and the non-skin-side surface of the absorbent article 1. For example, it is preferable if the chemical-carrying portion A is provided in a place separate from the skin-side surface (the inner surface) of the inner surface sheet 3 and the non-skin-side surface (the outer surface) of the outer surface sheet 2. It is preferable not to place the chemical-carrying portion A on the skin-side (inner) exposed surface of the absorbent article 1 because the chemical is thereby prevented from directly contacting the skin. It is also preferable not to place the chemical-carrying portion A on the non-skin-side (outer) exposed surface of the absorbent article 1 because the chemical is thereby prevented from contacting the underwear when the absorbent article 1 is in use, and because this does not impair the function of the adhesive portions that are provided on the non-skin-side surface of the outer surface sheet 2 in many cases.

The chemical-carrying portion A may be formed, for example, by applying the chemical to one or more of the surface of the absorber 4 on the inner surface sheet 3 side, the inside of the absorber 4, and the surface of the absorber 4 on the outer surface sheet 2 side. The chemical may be also applied to an end surface of the absorber 4 if the absorber 4 is thick. When the absorber 4 is wrapped in the wrapping sheet 5 as mentioned earlier, the chemical-carrying portion A may be formed on the outer surface of the wrapping sheet 5 (the surface opposite to the surface facing the absorber 4), or may be formed between the absorber 4 and the wrapping sheet 5 by, for example, applying the chemical to the surface of the wrapping sheet 5 facing the absorber 4. Furthermore, the chemical-carrying portion A may be provided on the non-skin-side surface of the inner surface sheet 3 or the skin-side surface of the outer surface sheet 2. Furthermore, in the event a separate sheet is provided between the inner surface sheet 3 and the absorber 4 or between the absorber 4 and the outer surface sheet 2, the chemical-carrying portion A may be provided in that separate sheet. For example, a second sheet can be provided between the inner surface sheet 3 and the absorber 4. In that case, the chemical-carrying portion A can be provided on the inner surface side or the outer surface side of the second sheet.

The chemical-carrying portion A may be formed in one or more of the above-mentioned locations, that is, in multiple different locations in the thickness direction. Note that, from the viewpoint of allowing the person who wears the absorbent article 1 to notice the effects of the chemical more, the chemical-carrying portion A is preferably formed by applying the chemical to one or more of the non-skin-side surface of the inner surface sheet 3 and the skin-side surface of the absorber 4.

Furthermore, the chemical-carrying portion A may be provided in the front area F or in the rear area R, or in both the front area F and the rear area R. By this means, the chemical-carrying portion A can be provided in a place that is at a distance from the body fluid discharging orifice facing area Q, and the possibility that body fluids come into contact with the chemical can be reduced. The chemical-carrying portion A may be provided in the rear area R, for example, as illustrated in FIG. 1.

Although an additional chemical-carrying portion A can be provided in the middle area M too, the amount of the chemical to provide in the middle area M is preferably smaller than the amount of the chemical provided in the front area F and the rear area R. Furthermore, in the total amount of the chemical that is applied to the absorbent article 1, the amount of the chemical to be provided in at least one of the front area F and the rear area R is preferably as large as possible, and the amount of the chemical to be provided in the middle area M is preferably as small as possible. To be more specific, it is more preferable if the amount of the chemical that is applied to the front area F and the rear area R is 95% or more of the total amount of the chemical applied to the absorbent article 1, and the amount of the chemical that is applied to the middle area M is 5% or less of the total amount of the chemical applied to the absorbent article 1.

The amount of the chemical to be applied may be preferably 10 g/m² to 100 g/m², more preferably 30 g/m² to 70 g/m². Furthermore, the area of the chemical-carrying portion A in plan view may be preferably 100 mm² to 2500 mm², more preferably 400 mm² to 1600 mm². Furthermore, the plan-view shape of the chemical-carrying portion A is not particularly limited, and may be a quadrangle as illustrated in FIG. 1, or may be a polygon other than the quadrangle, a circle, an ellipse, or the like. Furthermore, the range for applying the chemical is not particularly limited either. In order to reduce the possibility of impairing the function of the adhesive or the like that is used to join the parts of the absorbent article 1, it is preferable for the chemical-carrying portion A to not overlap with the joint portion of the inner surface sheet 3 and the outer surface sheet 2, the joint portion of the inner surface sheet 3 and the side non-woven fabrics 7, or the like.

The type of the chemical to be used is not limited, but a liquid chemical, in which a functional element that can impart some function to the absorbent article 1 is dispersed or dissolved in a solvent, is preferable. The functional element may include, for example, one or more of a fragrance element, a deodorant element, a refreshing sensation improving element, warm sensation improving element, and the like, and is preferably volatile.

The fragrance element may be a natural fragrance or a synthetic fragrance. Specific examples of natural fragrances include ambergris, benzoin, castoreum, civet, clove oil, galbanum, jasmine absolute, labdanum, mate tea, melilot, mimosa, musk tonquin, myrrh, oakmoss or mousse de chene, frankincense, byakushi scent, orris, patchouli, rosemary oil, ebony oil, vetiver oil, violet leaf absolute, and the like. Specific examples of synthetic fragrances include higher alcohol, aldehyde, benzaldehyde, benzoic acid, cinnamic acid, cinnamic aldehyde, cinnamic alcohol, coumarin, ester, indole, ketone, salicylic acid and related compounds, terpenoid, vanillin, and the like.

As for the functional element to be contained in the chemical, two or more types of elements may be combined and contained. Furthermore, an element having a deodorizing effect as well as an aromatic effect can be used. Furthermore, the functional element to be contained in the chemical may be in the form of an inclusion compound that is enveloped by a host compound, or may be in a form that is micro-encapsulated with use of a membrane material, depending on the solvent and the type of functional element.

Since the chemical that is applied is easy to handle and its rate of volatilization can be adjusted as appropriate, the chemical, upon its application, is preferably in the state of a solution in which the functional element is dissolved by using a solvent of an aqueous substance, especially water.

The means for applying the chemical is not particularly limited. The chemical can be applied by using, for example, a sprayer, a brush, a bar coater, a gravure coater, a roll coater, inkjet printing, and so forth. Of these, a sprayer, which is a means to allow a chemical to fly in midair as droplets and land, is particularly preferable because even a small amount of chemical can be applied uniformly.

### (Compressed portions)

As illustrated in FIG. 1 and FIG. 2, the absorbent article 1 may be provided with compressed portions 10. A compressed portion 10 is a portion that is subjected to a process (embossing) of partially applying pressure from the skin side (the inner surface sheet 3 side) to the non-skin side (the outer surface sheet 2 side) to increase the density of the portion (this process if also referred to as "fit embossing"). The compressed portions 10 can be formed by compressing, from the skin side, a laminate that is formed by laminating the absorber 4 and the inner surface sheet 3, or a laminate formed by laminating the absorber 4, the second sheet, and the inner surface sheet 3. For example, the compressed portions 10 can be formed by passing the above-mentioned laminate between a pair of press rolls. In that case, the laminate is arranged such that a convex roll meets the skin-side surface of the laminate and a flat roll meets the non-skin-side surface of the laminate.

While the shape of the compressed portions 10 is not limited and the compressed portions 10 may be shaped like dots, as will be described later, providing the compressed portions 10 in the form of a line or a groove is preferable. Such linear or groove-shaped compressed portions 10 are formed to avoid crossing the body fluid discharging orifice facing area Q, but are not limited to any particular plan-view shape. However, at least, the compressed portion 10 may have a pair of front and rear compressed portions in the longitudinal direction, which pass through the middle area M and are provided on both sides of the longitudinal center line CL. Furthermore, individual compressed portions 10 may be provided in the front area F and the rear area R.

The compressed portions 10 may have a compressed portion 11 that serves to block incoming body fluids. This "blocking" compressed portion 11 is formed at a distance from the chemical-carrying portion A, on a straight line connecting the center Q_{C} of the body fluid discharging orifice facing area Q with the chemical-carrying portion A. In other words, the chemical-carrying portion A may be provided, in plan view, on a straight line that passes through the center Q_{C} of the body fluid discharging orifice facing area Q and the blocking compressed portion 11. Furthermore, it is possible to say that all the straight lines connecting the locations of the chemical-carrying portion A and the center Q_{C} of the body fluid discharging orifice facing area Q pass through the compressed portion provided at a distance from the chemical-carrying portion A between the chemical-carrying portion A and the center QC of the body fluid discharging orifice facing area Q. In addition, in this specification, compressed portions other than the blocking compressed portion 11 may be each simply referred to as "compressed portions 12."

While the absorbent article 1 is in use, usually, a body fluid is discharged to the body fluid discharging orifice facing area Q. However, the body fluid might later move and diffuse outwards from the body fluid discharging orifice facing area Q inside and on the surface of the absorbent article 1. Especially, if the front and rear compressed portions are provided as described above, the body fluid moves forward and/or rearward more readily. Here, some chemicals used in the chemical-carrying portion A might change their properties when they contact body fluids. In particular, when a chemical contains an aromatic element, its function often deteriorates by mixing with body fluids, and, depending on the user, the odor of the mixture of the aromatic element with body fluids may be perceived as an unpleasant odor. Therefore, while the absorbent article 1 is in use, it is preferable to prevent incoming body fluids from contacting the chemical-carrying portion A as much as possible.

According to the present embodiment, the blocking compressed portion 11 is formed at a distance from the chemical-carrying portion A, on a straight line that connects the center Q_{C} of the body fluid discharging orifice facing area Q and the chemical-carrying portion A. As a result of this, the body fluid that comes from the body fluid discharging orifice facing area Q might arrive at the blocking compressed portion 11 before arriving at the chemical-carrying portion A. Provided that the compressed portion is a densified portion as described above, the likelihood is high that the body fluid is guided to the compressed portion and held in the compressed portion. Furthermore, even when the body fluid cannot be held in the compressed portion, such as when a large amount of body fluid is discharged, the route on which the body fluid moves can be changed to be longer. Consequently, the movement of the body fluid is stopped, at least temporarily, by the blocking compressed portion 11, which can prevent or delay the arrival of the body fluid at the chemical-carrying portion A.

Note that, if the compressed portion 12 is not connected with the blocking compressed portion 11, for example, as illustrated in FIG. 1, the compressed portion 12 and the chemical-carrying portion A may overlap in plan view. The form in which the chemical-carrying portion A and the compressed portion 12 overlap can be obtained by applying the chemical to the densified portion. This makes it easy to gather and hold the body fluid in the compressed portion, so that the chemical volatilizes little, and the function of the chemical can be maintained for a longer period of time.

### (Modifications)

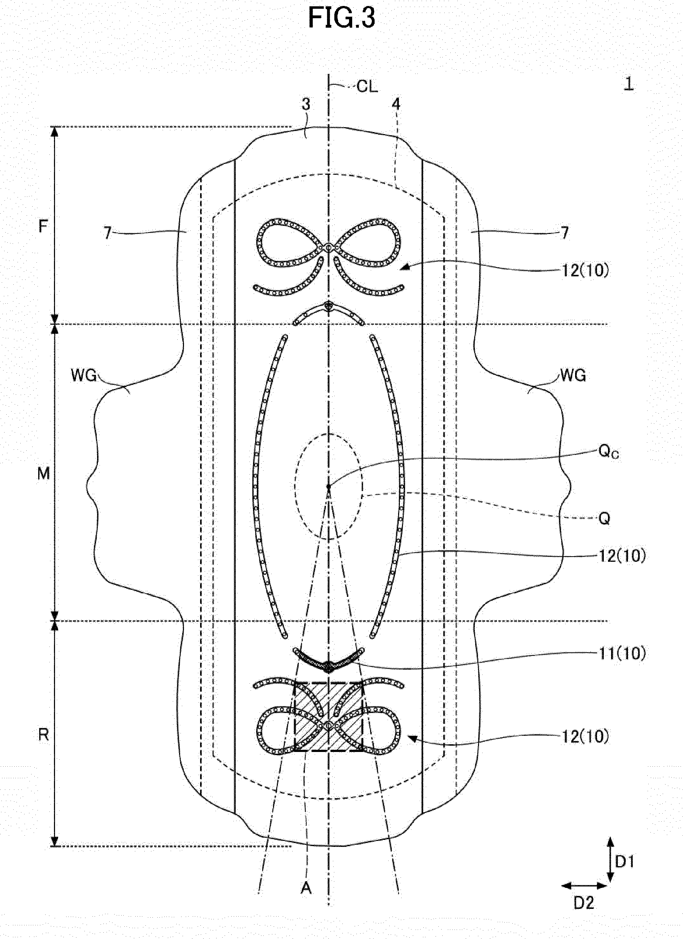

FIG. 3 illustrates a modification of the present invention. The absorbent article 1 illustrated in FIG. 3 has the same basic structure as the absorbent article 1 illustrated in FIG. 1, but the shape of the compressed portions 10 is different. In the example of FIG. 3, again, a blocking compressed portion 11 is formed at a distance from the chemical-carrying portion A, on a straight line that connects the center Q_{C} of the body fluid discharging orifice facing area Q and the chemical-carrying portion A, but the compressed portion 10 including the blocking compressed portion 11 is at a distance from the pair of front and rear compressed portions formed in the middle area M and extending in the longitudinal direction D1.

In all of the examples illustrated in FIG. 1 to FIG. 3, the groove-shaped blocking compressed portion 11 intersects the longitudinal center line CL. That is, a groove-shaped blocking compressed portion 11 is provided at an angle with respect to the longitudinal center line CL, or extends in a direction including an element of the width direction D2. As a result of this, the body fluid that comes forward and/or backward hits the edge portion of the groove-shaped blocking compressed portion 11, and thus the body fluid can be blocked by a wider width, and the blocking effect improves.

Furthermore, the groove-shaped blocking compressed portion 11 does not necessarily have to intersect the longitudinal center line CL, and an extension line of the groove-shaped blocking compressed portion 11 may intersect the longitudinal center line CL.

The groove-shaped blocking compressed portion 11 may be linear or curved, but a curve is more preferable. Furthermore, in the event the blocking compressed portion 11 has a shape that is outwardly convex in the longitudinal direction D1 (that is, convex in a direction to go away from the body fluid discharging orifice facing area Q), when a large amount of body fluid suddenly moves forward or rearward, it is still easy to block the body fluid on the inner side of the blocking compressed portion 11 in the longitudinal direction D1, and make it difficult for the body fluid to arrive at the chemical-carrying portion A.

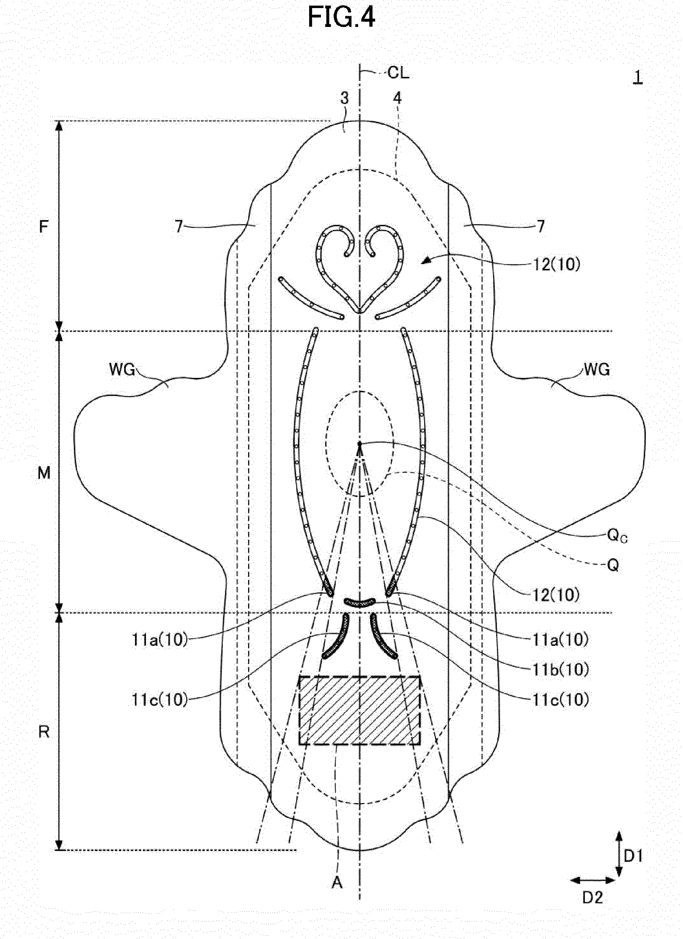

FIG. 4 illustrates another modification of the present invention. In the example illustrated in FIG. 4, the basic structure, which is formed by laminating the outer surface sheet 2, the absorber 4, and the inner surface sheet 3, is the same as the examples illustrated in FIG. 1 to FIG. 3, but the contour of the absorbent article 1, the shape of the absorber 4, and the configuration of the compressed portion 10 are different. In the example of FIG. 4, again, the compressed portions 10 include the blocking compressed portion 11, except that multiple compressed portions 11 are provided in this example. The blocking compressed portions 11 have: a pair of blocking compressed portions 11a and 11a, which are formed as rear end portions of a pair of front and rear compression portions formed in the middle area M following the longitudinal direction D1; a blocking compressed portion 11b, which intersects the longitudinal center line CL, and which is curved convexly rearward; and a pair of blocking compressed portions 11c and 11c, which are formed behind the blocking compressed portion 11b, at a distance from each other in the width direction D2, and in which the separating width between the pair increases rearward.

The blocking compressed portions 11a and 11a, 11b, and 11c and 11c are provided at a distance from one another, each formed at a distance from the chemical-carrying portion A, on a straight line that connects the center Q_{C} of the body fluid discharging orifice facing area Q and the chemical-carrying portion A. In other words, a straight line that extends radially rearward from the center Q_{C} of the body fluid discharging orifice facing area Q passes by one of the blocking compressed portions 11 before arriving at the chemical-carrying portion A. Consequently, even if a blocking compressed portion 11 that is contiguous in the width direction D2 is not formed, it is still possible to exert a blocking effect for the chemical-carrying portion A that is formed large in the width direction D2, by combining multiple separate blocking compressed portions 11 as illustrated in FIG. 4.

Furthermore, referring to the example of FIG. 4, there are portions where a number of blocking compressed portions 11 are formed on a straight line connecting the center Q_{C} of the body fluid discharging orifice facing area Q and the chemical-carrying portion A. To be more specific, among the straight lines connecting the center Q_{C} of the body fluid discharging orifice facing area Q and the chemical-carrying portion A, there are straight lines that intersect both of the blocking compressed portion 11b and the blocking compressed portion 11c. In this way, when a straight line connecting the center Q_{C} of the body fluid discharging orifice facing area Q and the chemical-carrying portion A intersects multiple blocking compressed portions 11, this is preferable because the blocking compressed portions 11 provide an improved blocking effect.

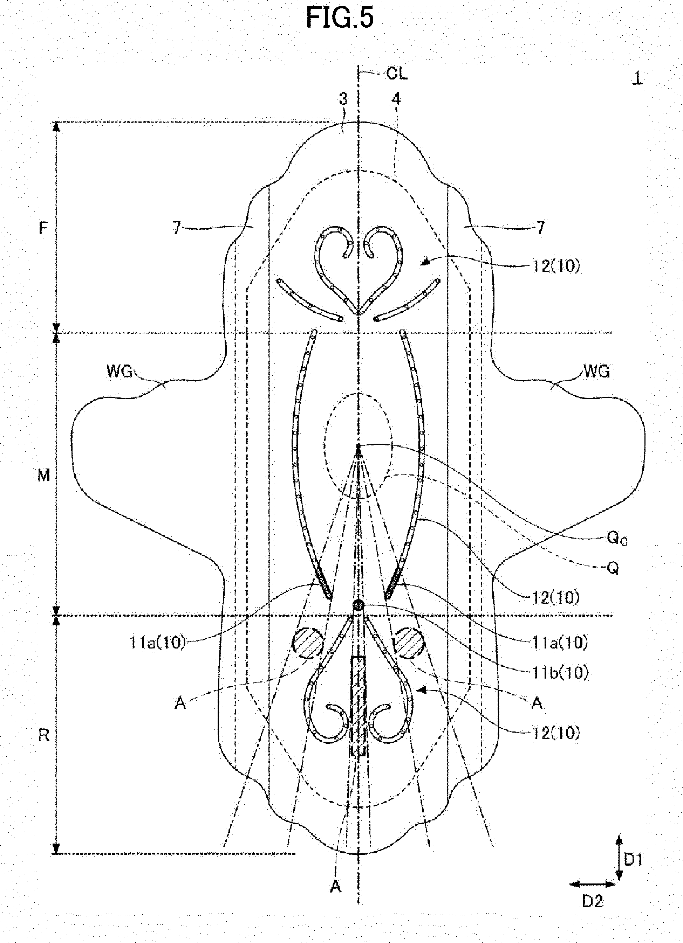

FIG. 5 illustrates another modification of the present invention. In the example illustrated in FIG. 5, the basic structure formed by laminating the outer surface sheet 2, the absorber 4, and the inner surface sheet 3, the contour of the absorbent article 1, and the shape of the absorber 4 are the same as the example illustrated in FIG. 4, but the configurations of the compressed portions 10 and the chemical-carrying portion A are different. In the example of FIG. 5, again, the compressed portions 10 include multiple blocking compressed portions 11, and the blocking compressed portions 11 include a pair of blocking compressed portions 11a and 11a, which are formed as rear end portions of a pair of front and rear compressed portions formed in the middle area M following the longitudinal direction D1, and a dot-shaped blocking compressed portion 11b provided on the longitudinal center line CL.

The blocking compressed portions 11a and 11a and 11b are each provided at a distance from the chemical-carrying portion A, on a straight line that connects the center Q_{C} of the body fluid discharging orifice facing area Q and the chemical-carrying portion A. In other words, the chemical-carrying portion A is formed such that every location of the chemical-carrying portion A is provided on a straight line that starts from the center Q_{C} of the body fluid discharging orifice facing area Q and passes by one of the blocking compressed portions 11a and 11a and 11b. For example, the blocking compressed portions 11a and 11a and the dot-shaped blocking compressed portion 11b are at a distance from each other, and the chemical-carrying portion A is not provided on any of the straight lines that extend radially rearward from the center Q_{C} of the body fluid discharging orifice facing area Q. In this way, in the example of FIG. 5, the chemical-carrying portion A is formed in multiple pieces in accordance with the locations of the blocking compressed portions 11a and 11a and the dot-shaped blocking compressed portion 11b.

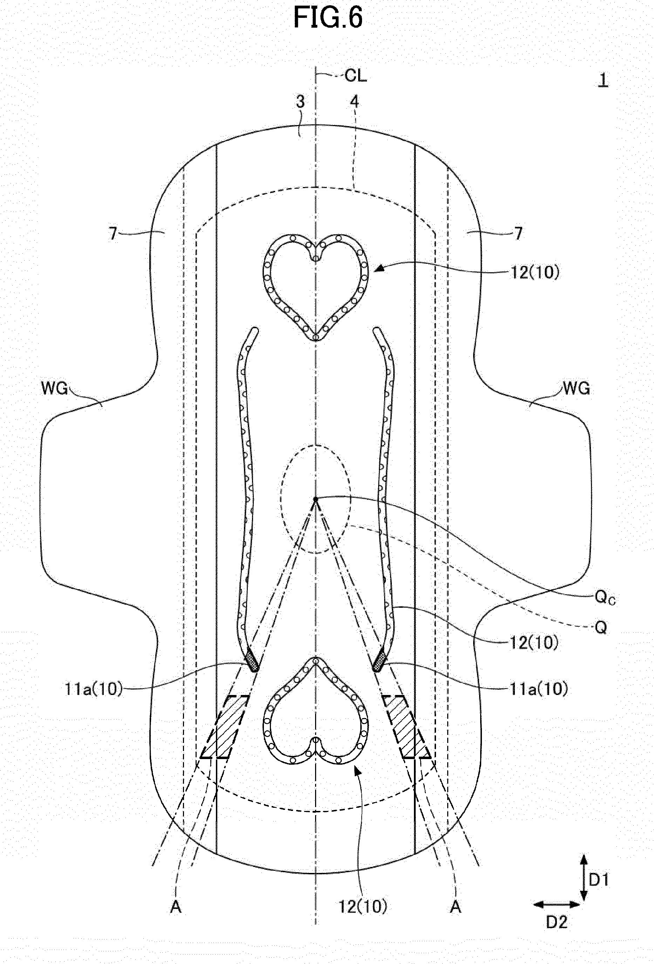

FIG. 6 illustrates another modification of the present invention. In the example illustrated in FIG. 6, the basic structure formed by laminating the outer surface sheet 2, the absorber 4, and the inner surface sheet 3 is the same as in the examples illustrated in FIG. 1 to FIG. 5, but the contour of the absorbent article 1, the shape of the absorber 4, and the configurations of the compressed portions 10 and the chemical applying portion A are different. In the example of FIG. 6, again, the compressed portions 10 include multiple blocking compressed portions 11, but the blocking compressed portions 11 are composed only of a pair of blocking compressed portions 11a and 11a, which are formed as rear end portions of a pair of front and rear compressed portions formed in the middle area M following the longitudinal direction D1.

The pair of blocking compressed portions 11a and 11a are each provided at a distance from the chemical-carrying portion A, on a straight line that connects the center Q_{C} of the body fluid discharging orifice facing area Q and the chemical-carrying portion A. In the example of FIG. 6, two chemical-carrying portions A are formed on each side of the longitudinal center line CL. Furthermore, each chemical-carrying portion A is formed within a range that is formed by straight lines starting from the center Q_{C} of the body fluid discharging orifice facing area Q and passing by one of the blocking compressed portions 11a and 11a.

In particular, when a large amount of body fluid is suddenly discharged, or when the user is in the supine position, the body fluid tends to move and permeate in the center of the rear area R in the width direction D2. Therefore, as illustrated in the example of FIG. 6, if no chemical-carrying portion A is formed on the longitudinal center line CL, or, to be more specific, if no chemical-carrying portion A is formed in the center of the width direction D2, and yet multiple chemical-carrying portions A are formed at a distance from each other in the width direction D2, the possibility that the body fluids contact the chemical can be reduced. Therefore, the chemical does not get mixed with body fluids, and the blocking compressed portions can exert their function more reliably.

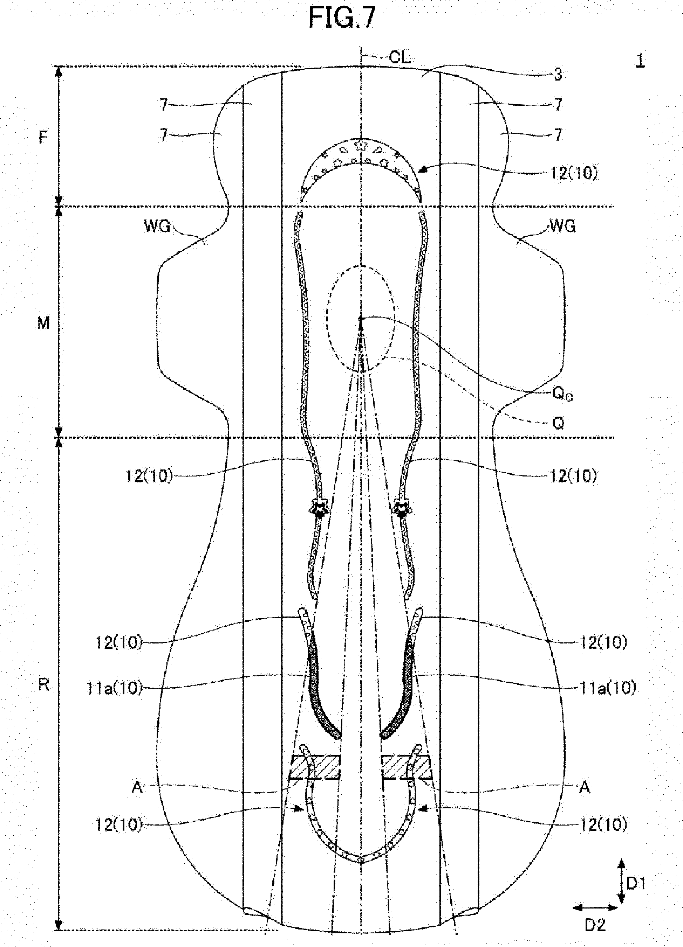

FIG. 7 also illustrates another modification of the present invention. The absorbent article 1 illustrated in FIG. 7 is suitable for use as a sanitary napkin for night use or for use over a long period of time, having a long overall length. In the example illustrated in FIG. 7, again, the basic structure, which is formed by laminating the outer surface sheet 2, the absorber 4, and the inner surface sheet 3, is the same as the examples illustrated in FIG. 1 to FIG. 6, but the contour of the absorbent article 1, the configuration of the non-woven fabric 7, and the like are different. Furthermore, the configurations of the compressed portions 10 and the chemical-carrying portion A are different.

Similar to the compressed portions 10 in FIG. 6, the compressed portions 10 in the example of FIG. 7 include a pair of blocking compressed portions 11a and 11a, which are formed as rear end portions of a pair of front and rear compressed portions formed in the middle area M following the longitudinal direction D1. Each chemical-carrying portion A is formed within a range that is formed by straight lines starting from the center Q_{C} of the body fluid discharging orifice facing area Q and passing by one of the blocking compressed portions 11a and 11a. That is, in the example of FIG. 7, the chemical-carrying portions A are provided as two chemical-carrying portions A and A, formed on both sides of the longitudinal center line CL.

As described above, in all of the examples of FIG. 1 to FIG. 7, each blocking compressed portion 11 is formed at a distance from the chemical-carrying portion A, on a straight line that connects the center Q_{C} of the body fluid discharging orifice facing area Q and the chemical-carrying portion A. Consequently, when a body fluid that is discharged mainly in the body fluid discharging orifice facing area Q comes forward and/or rearward, the blocking compressed portions 11 can block or delay the movement of the body fluid, so that the contact of the body fluid and the chemical-carrying portion A can be prevented or reduced. Therefore, the function of the chemical contained in the chemical-carrying portion A can be maintained.

### (Examples of compressed portions formed in the absorber)

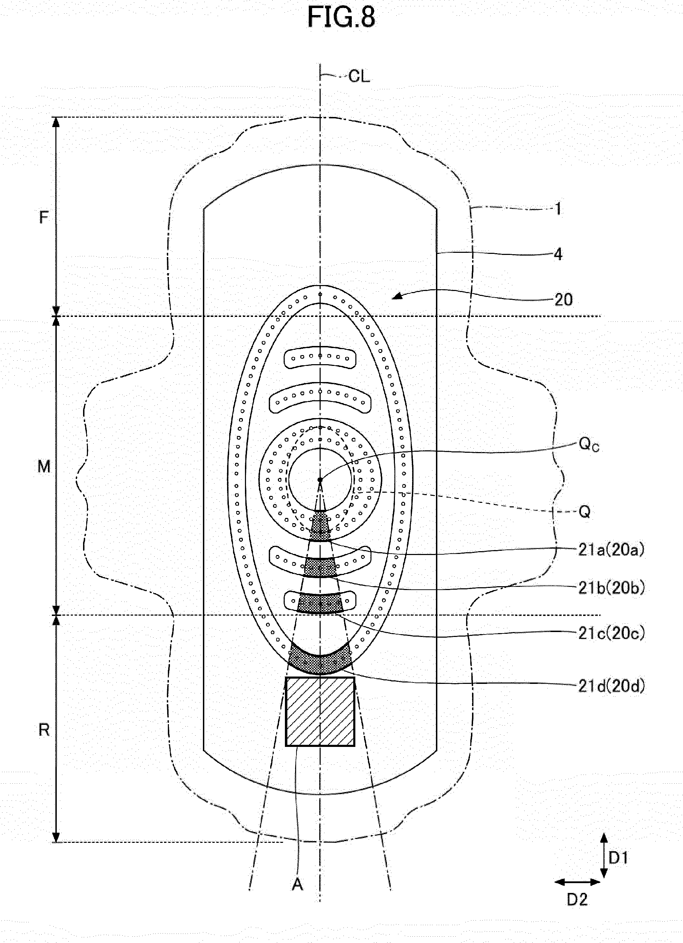

FIG. 8 illustrates a plan view of the absorber 4 used in the absorbent article 1 illustrated in FIG. 1. The absorber 4 illustrated in FIG. 8 is in a state before the inner surface sheet 3 is laminated, that is, before the laminate of the absorber 4 and the inner surface sheet 3 is compressed and the compressed portions 10 are formed. As illustrated in FIG. 8, compressed portions 20 are formed in the absorber 4 as well.

Each compressed portion 20 is a portion (also referred to as "core embossing") of the absorber 4 subjected to a process of applying pressure partially from the skin side (the inner surface sheet 3 side) to the non-skin side (the outer surface sheet 2 side), to increase the density of the portion. The compressed portions 20 can be formed by compressing the absorber 4 from the skin side before or after the absorber 4 is wrapped in the wrapping sheet 5. For example, the compressed portions 20 can be formed by passing the absorber 4 between a pair of press rolls. In that case, the absorber 4 is arranged such that a convex roll meets the skin-side surface of the absorber 4 and a flat roll meets the non-skin-side surface of the absorber 4. The compressed portions 20 formed in the absorber 4 may have the same plan-view shape as the compressed portions 10 formed in the laminate including the absorber 4 and the inner surface sheet 3, or may have a different plan-view shape.

As illustrated in FIG. 8, the compressed portions (core embossing) 20 may have an annular compressed portion 20a that is concentric with the center Q_{C} of the body fluid discharging orifice facing area Q, an elliptical compressed portion 20d that is formed around the annular compressed portion 20a, and compressed portions 20b and 20c that are formed between these, pass through the longitudinal center line CL, and are slightly convex outwards in the longitudinal direction D1 (in a direction to go away from the body fluid discharging orifice facing area Q). The compressed portion 20d might overlap a compressed portion (fit embossing) 10 (FIG. 1) in plan view, but the compressed portions 20a, 20b, and 20c do not overlap the compressed portions (fit embossing) 10 (FIG. 1) in plan view, and their locations cannot be readily identified visually or are difficult to identify visually, only by looking at the outward look of the absorbent article 1.

The compressed portions (core embossing) 20 also have blocking compressed portions 21. The blocking and compressed portions 21 have the same configuration as the blocking compressed portions 11 of the above-mentioned compressed portions (fit embossing) 10, and provide the same function and effect. That is, each blocking compressed portion 21 is formed at a distance from the chemical-carrying portion A, on a straight line that connects the center Q_{C} of the body fluid discharging orifice facing area Q and the chemical-carrying portion A,. By this means, when a body fluid arrives from the body fluid discharging orifice facing area Q, the arriving body fluid can be blocked on the front side of the chemical-carrying portion A, thereby preventing or reducing the contact of the body fluid with the chemical.

The blocking compressed portion 21 may have blocking compressed portions 21a, 21b, 21c, and 21d that intersect the longitudinal center line CL and are provided at a distance from each other in the longitudinal direction D1. The blocking compressed portions 21a, 21b, 21c, and 21d are parts of the compressed portions 20a, 20b, 20c, and 20d, respectively.

When both compressed portions (fit embossing) 10 and compressed portions (core embossing) 20 are formed in the absorbent article 1, it suffices if either the compressed portions (fit embossing) 10 or the compressed portions (core embossing) 20 satisfy the requirement that each compressed portion be formed at a distance from the chemical-carrying portion A, on a straight line that connects the center QC of the body fluid discharging orifice facing area Q and the chemical-carrying portion A. However, it is more preferable if both compressed portions (fit embossing) 10 and compressed portions (core embossing) 20 satisfy the above requirement.

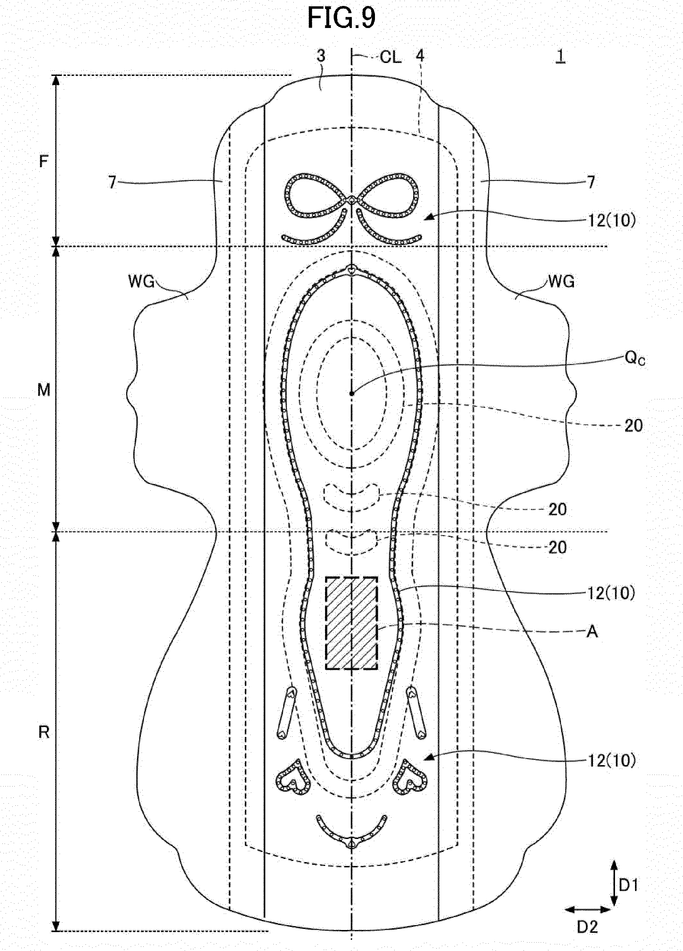

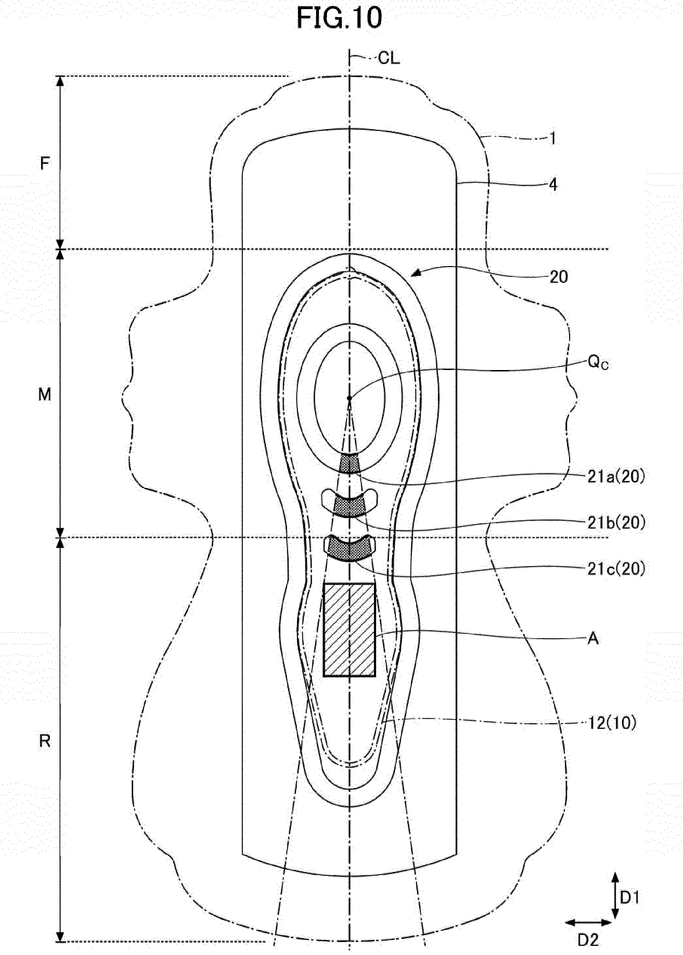

FIG. 9 and FIG. 10 illustrate another modification of the absorbent article 1, in which both compressed portions (fit embossing) 10 and compressed portions (core embossing) 20 are formed. FIG. 9 illustrates a plan view of the absorbent article 1 as viewed from the inner surface sheet 3 side in an unfolded state. FIG. 10 illustrates a plan view of the absorber 4 included in FIG. 9, in a state before being laminated with other components.

As illustrated in FIG. 9, the absorbent article 1 is formed with a chemical-carrying portion A and compressed portions (fit embossing) 10, compressed from the inner surface sheet 3 side in a state in which the absorber 4 and the inner surface sheet 3 are laminated. The compressed portions 10 are not formed on a straight line connecting the center Q_{C} of the body fluid discharging orifice facing area Q and the chemical-carrying portion A. However, as illustrated in FIG. 10 (or as shown by dotted lines in FIG. 9), the compressed portions (core embossing) 20 are formed in the absorber 4. The compressed portions 20 have blocking compressed portions 21a, 21b, and 21c, which are each formed at a distance from the chemical-carrying portion A, on a straight line that connects the center QC of the body fluid discharging orifice facing area Q and the chemical-carrying portion A. As described above, although the compressed portions (fit embossing) 10 in the example illustrated in FIG. 9 and FIG. 10 do not satisfy the requirement that each compressed portion be formed at a distance from the chemical-carrying portion A on a straight line that connects the center Q_{C} of the body fluid discharging orifice facing area Q and the chemical-carrying portion A, the compressed portions (core embossing) 20 satisfy this requirement, so that the effect of blocking body fluids can be achieved, and the function of the chemical can be maintained.

Note that, in this example, multiple blocking compressed portions, or, to be more specific, three blocking compressed portions 21a, 21b, and 21c, are each formed on a straight line that connects the center Q_{C} of the body fluid discharging orifice facing area Q and the chemical-carrying portion A. Therefore, the effect of blocking body fluids is excellent.

### (Wrapped absorbent article)

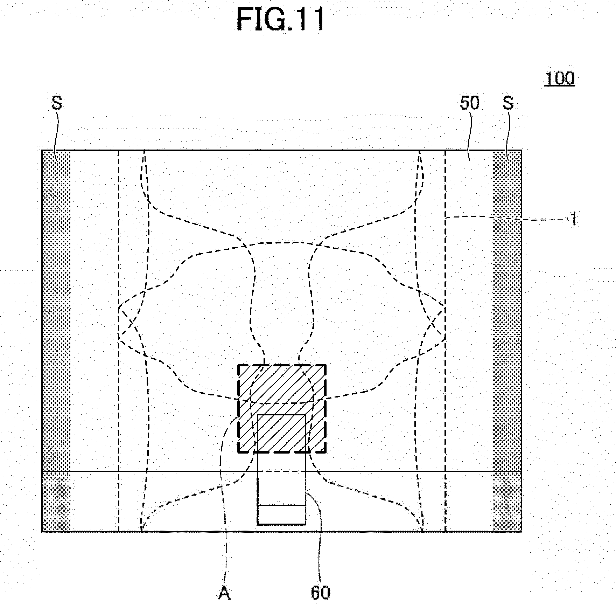

FIG. 11 shows a wrapped absorbent article 100 obtained by folding and wrapping the absorbent article 1 illustrated in FIG. 1 with a wrapping sheet 50. To wrap the absorbent article 1 with the wrapping sheet 50, as illustrated in FIG. 1, the absorbent article 1 is placed on the wrapping sheet 50, and the wrapping sheet 50 and the absorbent article 1 are folded together in the longitudinal direction D1, to the inner surface sheet 3 side (to the skin side). After the rear area R is folded back at a first fold line L1, the front area F is folded back at a second fold line L2 so that the front area F is piled on the rear area R (FIG. 1). Note that it is preferable to have the wings WG and WG folded back towards the inner surface sheet 3 side in advance before the above fold in the longitudinal direction D1.

At both edges of the wrapping sheet 50 in the width direction D2, sealing portions S and S can be formed which, after the wrapping sheet 50 and the absorbent article 1 are folded, can be sealed by applying heat and/or pressure and unsealed (FIG. 11). The sealing portions S and S may be formed by, for example, heat sealing, ultrasonic sealing, mechanical sealing by embossing without melting of the wrapping sheet, and the like. Also, the sealing portions S and S may also be formed by using an adhesive. Furthermore, at the center of the width direction D2, a fastening tape 60 may be applied from the front area F to a portion of the rear area F that is not piled on the front area F.

When the rear area R is folded before the front area F is folded, the rear area R is placed further inside the wrapped absorbent article 100. Therefore, by forming the chemical-carrying portion A in the rear area R in advance, the chemical volatilizes less before the absorbent article 100 is opened. By this means, even if the time from the manufacturing of the wrapped-absorbent article 100 until it is opened is long, the function of the chemical is not impaired significantly.

Although specific examples of the present invention have been described above by using a sanitary napkin as an example, the present invention can be a panty liner, an incontinence pad, and the like. However, since many chemicals tend to deteriorate in function when mixed with menstrual blood, the present invention is particularly suitable for use as a sanitary napkin.

Now, specific aspects of the present invention will be noted below.

### (Note 1)

An aspect of the present invention according to note 1 provides an absorbent article that includes: a liquid-impermeable outer surface sheet; an absorber; and a liquid-permeable inner surface sheet. These are laminated in the order named. The absorbent article has a longitudinal direction that corresponds to the longitudinal direction of the body of the user of the absorbent article, and a width direction that is orthogonal to the longitudinal direction. The absorbent article has a middle area that includes a body fluid discharging orifice facing area facing a body fluid discharging orifice in the body of the person, a front area that is joined to a front portion of the middle area, and a rear area that is joined to a rear portion of the middle area. A chemical-carrying portion is formed in at least one of the front area and the rear area of the absorbent article, the chemical-carrying portion being where a chemical is applied to a surface that is separate from an exposed surface of the absorbent article. A compressed portion is provided at a distance from the chemical-carrying portion, on a straight line that connects the center of the body fluid discharging orifice facing area and the chemical-carrying portion.

In this aspect of the present invention according to note 1, the compressed portion is provided on a straight line that connects the center of the body fluid discharging orifice facing area and the chemical-carrying portion. Therefore, while the absorbent article is in use, even if a body fluid received in the body fluid discharging orifice facing area moves linearly, for example, to the outside of the body fluid discharging orifice facing area, the body fluid arrives at the compressed portion before it arrives at the chemical-carrying portion, and therefore the body fluid is gathered and held in the compressed portion more readily through capillary action. In other words, the compressed portion can block the body fluid in front of the chemical-carrying portion. It then becomes difficult or takes time for the body fluid to contact the chemical-carrying portion, thereby preventing the chemical from contacting the body fluid and having its function deteriorated. Furthermore, since the compressed portion is formed at a distance from the chemical-carrying portion, it is possible to prevent the body fluid from moving along the compressed portion and arriving at the chemical-carrying portion.

### (Note 2)

In an aspect of the present invention according to note 2, the compressed portion includes a compressed portion that is provided in the shape of a groove, and the groove-shaped compressed portion or an extension line of the groove-shaped compressed portion intersects a center line that runs along the longitudinal direction.

In this aspect of the present invention according to note 2, the groove-shaped compressed portion forms an angle with respect to the longitudinal center line, that is, includes an element of the width direction. Therefore, the compressed portion can improve the effect of blocking the body fluid that comes forward and/or rearward from the body fluid discharging orifice facing area.

### (Note 3)

In an aspect of the present invention according to note 3, the groove-shaped compressed portion includes a convex portion that is convex towards the chemical-carrying portion and that intersects the center line that runs along the longitudinal direction.

In this aspect of the present invention according to note 3, a convex groove-shaped compressed portion is formed on the longitudinal center line of the absorbent article, that is, at the center of the absorbent article in the width direction, so that it is possible to block the body fluid that comes forward and/or rearward in the vicinity of the center of the absorbent article in the width direction more reliably. When the absorbent article is worn in a normal way, the body fluid tends to move from the body fluid discharging orifice facing area and pass the center in the width direction, and therefore, according to this example, the effect of blocking the body fluid by the compressed portion is improved.

### (Note 4)

In an aspect of the present invention according to note 4, the chemical-carrying portion is formed in the rear area.

In this aspect of the present invention according to note 4, when the absorbent article is folded and wrapped, the rear area of the absorbent article is placed inside the wrapped absorbent article for most of the time, so that, if the chemical is applied to that rear area in advance, the chemical does not readily volatilize before the absorbent article is opened. Also, when the absorbent article is in use, the rear area is susceptible to body forces and tends to expand and contract often, so that, depending on the purpose of use of the absorbent article or the type of the chemical, the volatilization of the chemical can be more readily facilitated, and the function of the chemical can be exerted more reliably.

### (Note 5)

In an aspect of the present invention according to note 5, the chemical-carrying portion includes portions that are formed at a distance from each other on both sides of the center line that runs along the longitudinal direction.

In this aspect of the present invention according to note 5, separate chemical-carrying portions are provided over the longitudinal center line, so that, even when a large amount of body fluid suddenly comes forward and/or rearward along the longitudinal center line, little body fluid contacts the chemical-carrying portion, and the function of the chemical can be exerted more reliably.

### (Note 6)

In an aspect of the present invention according to note 6, the amount of the chemical that is applied to the rear area and the front area is 95% or more of the total amount of the chemical that is applied to the absorbent article, and the amount of the chemical that is applied to the middle area is 5% or less of the total amount of the chemical that is applied to the absorbent article.

In this aspect of the present invention according to note 6, the amount of the chemical that is applied to the rear area and the front area is greater than or equal to a predetermined amount, and the amount of the chemical that is applied to the middle area including the body fluid discharging orifice facing area is less than or equal to the predetermined amount. Since the chemical is distributed unevenly in the rear area and/or the front area, the possibility that the body fluid gets mixed in the chemical-carrying portion can be further reduced.

### (Note 7)

An aspect of the present invention, according to note 7 provides a wrapped absorbent article that includes the absorbent article according to any one of above notes 1 to 6, and a wrapping sheet for that wraps the absorbent article.

In this aspect of the present invention according to note 7, it is possible to provide a wrapped absorbent article that provides the same effect as the effect according to any one of above notes 1 to 6.

### (Note 8)

In an aspect of the present invention according to note 8, the chemical-carrying portion is formed in the rear area, and, in a state in which the absorbent article is wrapped, the front area and the rear area of the absorbent article are folded with the wrapping sheet, in the longitudinal direction, towards the inner surface sheet, so that the front area is piled on the rear area.

In this aspect of the present invention according to note 8, the chemical-carrying area is formed in the rear area, and the absorbent article is folded so that, after the rear area is folded, the front area is piled on top of the rear area. By this means, the chemical-carrying area is placed inside the wrapped absorbent article, so that the chemical can be prevented from volatilizing before the absorbent article is opened.

This application is based on and claims priority to Japanese Patent Application No. 2020-055838, filed with Japan Patent Office on March 26, 2020, the entire contents of which are incorporated herein by reference.

### DESCRIPTION OF THE REFERENCE NUMERALS

1 Absorbent article
2 Outer surface sheet
3 Inner surface sheet
4 Absorber
7 Side non-woven fabric
10 Compressed portion (fit embossing)
11, 11a, 11b, 11c Fit-embossed blocking compressed portion
12 Compressed portion
20, 20a, 20b, 20c, 20d Compressed portion (core embossing) of the absorber
21, 21a, 21b, 21c, 21d Core-embossed blocking compressed portion
50 Wrapping sheet
60 Fastening tape
100 Wrapped absorbent article
A Chemical-carrying portion
CL Longitudinal center line
D1 First direction
D2 Second direction
F Front area of the absorbent article
L1 First fold line
L2 Second fold line
M Middle area of the absorbent article
R Rear area of the absorbent article
Q Body fluid discharging orifice facing area
Q_{C} Center of the body fluid discharging orifice facing area
S Sealing portion
WG Wing

## Claims

1. An absorbent article comprising a liquid-impermeable outer surface sheet, an absorber, and a liquid-permeable inner surface sheet that are laminated in order,
wherein the absorbent article has
a longitudinal direction that corresponds to a longitudinal direction of a body of a user of the absorbent article, and
a width direction that is orthogonal to the longitudinal direction,
wherein the absorbent article has
a middle area that includes a body fluid discharging orifice facing area facing a body fluid discharging orifice in the body of the person,
a front area that is joined to a front portion of the middle area, and
a rear area that is joined to a rear portion of the middle area,
wherein a chemical-carrying portion is formed in at least one of the front area and the rear area of the absorbent article, the chemical-carrying portion being where a chemical is applied to a surface that is separate from an exposed surface of the absorbent article, and
wherein a compressed portion is provided at a distance from the chemical-carrying portion, on a straight line that connects a center of the body fluid discharging orifice facing area and the chemical-carrying portion.

2. The absorbent article according to claim 1,
wherein the compressed portion includes a compressed portion that is provided in a shape of a groove, and
wherein the groove-shaped compressed portion or an extension line of the groove-shaped compressed portion intersects a center line that runs along the longitudinal direction.

3. The absorbent article according to claim 2, wherein the groove-shaped compressed portion includes a convex portion that is convex towards the chemical-carrying portion and that intersects the center line that runs along the longitudinal direction.

4. The absorbent article according to any one of claims 1 to 3, wherein the chemical-carrying portion is formed in the rear area.

5. The absorbent article according to any one of claims 1 to 4, wherein the chemical-carrying portion includes portions that are formed at a distance from each other on both sides of the center line that runs along the longitudinal direction.

6. The absorbent article according to any one of claims 1 to 5, wherein an amount of the chemical that is applied to the rear area and the front area is 95% or more of a total amount of the chemical that is applied to the absorbent article, and the amount of the chemical that is applied to the middle area is 5% or less of the total amount of the chemical that is applied to the absorbent article.

7. A wrapped absorbent article comprising the absorbent article according to any one of claims 1 to 6, and a wrapping sheet that wraps the absorbent article.

8. The wrapped absorbent article according to claim 7,
wherein the chemical-carrying portion is formed in the rear area, and
wherein, in a state in which the absorbent article is wrapped, the front area and the rear area of the absorbent article are folded with the wrapping sheet, in the longitudinal direction, towards the inner surface sheet, so that the front area is piled on the rear area.
